# DEMANDE DE BREVET EUROPEEN

(11) **EP 3 284 527 A1**
(43) Date de publication de la demande: **21.02.2018**
(21) Numéro de dépôt: 17184974.8
(22) Date de dépôt: 04.08.2017
(51) Int. Cl.: B01D 53/00, C12M 1/00, C10L 3/10

(54) **SYSTÈME DE TRAITEMENT D'UN GAZ**

(30) Priorité: 19.08.2016 FR 1657821
(71) Demandeur: Prodeval SAS, 26300 Chateauneuf-sur-Isère (FR)
(72) Inventeur: PEYRAT, Eric, 31100 TOULOUSE (FR); PAOLOZZI, Sébastien, 26300 SAINT-VINCENT-LA-COMMANDERIE (FR)
(74) Mandataire: Argyma

(57) **Abrégé**

L'invention concerne un procédé de traitement d'un flux principal de gaz. Ce procédé comprend une étape (E3) de prélèvement, dans le flux principal, d'un flux secondaire de gaz, une étape (E4) d'épuration du flux secondaire de gaz de sorte à produire, d'une part, un Gaz Naturel biologique pour Véhicule, et, d'autre part, des gaz résiduels, et une étape (E6) d'injection des gaz résiduels issus de l'épuration dans le flux principal de gaz.

## Description

### DOMAINE TECHNIQUE GENERAL

La présente invention se rapporte au domaine du traitement des gaz et concerne plus particulièrement un système et un procédé de traitement d'un gaz nécessitant une épuration. L'invention trouve notamment son application pour le traitement du biogaz issu de méthanisation ou des gaz de synthèse produits par méthanation et/ou pyrogazéification.

### ETAT DE LA TECHNIQUE

La méthanisation des matières organiques telles que, par exemple, les déchets organiques issus de l'agriculture, permet de produire du biogaz composé de méthane (CH₄) et de dioxyde de carbone (CO₂), par exemple environ à parts égales. Ce biogaz peut être utilisé de diverses manières. Ainsi, par exemple, il est connu de stocker le biogaz dans un gazomètre, de l'utiliser comme carburant dans un moteur ou une turbine pour produire de l'énergie électrique, de le bruler dans une chaudière pour produire de l'énergie thermique ou bien encore de l'épurer dans une unité d'épuration pour produire du Gaz Naturel biologique pour Véhicule, appelé BioGNV.

Dans une unité d'épuration, le rendement épuratoire est défini comme étant le rapport du débit de méthane sortant sur le débit de méthane entrant. Le rendement épuratoire est considéré comme élevé lorsqu'il est supérieur ou égal à 99 % et faible lorsqu'il est inférieur à 90 %. De manière connue, l'unité d'épuration permet d'appauvrir le biogaz en dioxyde de carbone de sorte qu'il soit constitué quasiment uniquement de méthane, par exemple de l'ordre de 97 %, afin de former du BioGNV. L'épuration du biogaz a pour effet de produire des gaz dits «résiduels» dont la composition dépend du rendement épuratoire de l'unité d'épuration. Ainsi, plus le rendement épuratoire est élevé, moins la composition des gaz résiduels comprend de méthane mais plus les coûts d'investissement et de fonctionnement de l'unité d'épuration sont élevés. Inversement, moins le rendement épuratoire est élevé, plus la composition des gaz résiduels comprend de méthane et plus les coûts d'investissement et de fonctionnement de l'unité d'épuration sont faibles. Aussi, afin de réduire les coûts, par exemple dans le cadre d'une exploitation agricole, il est nécessaire d'épurer le gaz avec un faible rendement épuratoire, quitte à produire moins de BioGNV. Ce faisant, l'utilisation du méthane contenu dans les gaz résiduels, dont la proportion augmente lorsque le rendement épuratoire diminue, n'est pas optimisée car les gaz résiduels sont soit détruits par oxydation thermique, soit brûlés dans des chaudières, soit rejetés dans l'atmosphère.

### PRESENTATION GENERALE DE L'INVENTION

L'invention a donc pour but de remédier à ces inconvénients en proposant une solution simple, fiable et efficace pour épurer un biogaz à faible rendement épuratoire tout en réduisant les pertes de méthane dans les gaz résiduels.

A cet effet, l'invention concerne tout d'abord un procédé de traitement d'un flux principal de gaz, ledit procédé étant remarquable en ce qu'il comprend :
- une étape de prélèvement, dans le flux principal, d'un flux secondaire de gaz,
- une étape d'épuration du flux secondaire de gaz de sorte à produire, d'une part, un Gaz Naturel biologique pour Véhicule, et, d'autre part, des gaz résiduels, et
- une étape d'injection des gaz résiduels issus de l'épuration dans le flux principal de gaz, qui a fait préalablement l'objet du prélèvement du flux secondaire de gaz.

Le procédé selon l'invention permet avantageusement de recycler le méthane contenu dans les gaz résiduels rejetés après l'épuration du gaz, notamment à faible rendement épuratoire. La présente invention permet notamment la valorisation in situ de la production excédentaire d'une installation de production de méthanisation à la ferme (dont la valorisation principale est la cogénération par exemple). Cette partie de fraction excédentaire est transformée par la présente invention en BioGNV sans perte de méthane, les gaz résiduels étant recyclés dans le circuit de valorisation du gaz. Sur cette fraction, la production de BioGNV permet par exemple l'alimentation d'une flotte de véhicules et d'engins utilisés par un agriculteur, lui apportant ainsi une autonomie énergétique. En exploitant intégralement la fraction excédentaire, la ressource répond bien au-delà de l'autonomie énergétique d'une exploitation agricole et peut s'étendre à des flottes captives extérieures à l'exploitation agricoles (engins industriels, chariots élévateurs, véhicules légers, camions de collecte de déchets, collecte laitière etc. ....). De préférence, le flux principal de gaz est constitué d'un biogaz issu d'une méthanisation et/ou d'un gaz de synthèse issu d'une méthanation ou d'une pyrogazéification.

Selon un aspect de l'invention, l'épuration du flux secondaire de gaz est réalisée à un rendement épuratoire inférieur à 90 %, de préférence compris entre 75 et 85 %, de manière à obtenir un taux de méthane suffisamment élevé pour valoriser le flux principal de gaz dans lequel ont été injectés les gaz résiduels.

Avantageusement, l'injection des gaz résiduels issus de l'épuration dans le flux principal de gaz est réalisée de sorte que le taux de méthane dans le flux principal après injection soit supérieur à 40 %. En effet, un taux de 40% de méthane dans un flux de gaz correspond au taux minimal accepté par la plupart des moteurs, torchères et chaudières conventionnels fonctionnant au gaz, ce qui permet d'utiliser du matériel de traitement standard de gaz ne nécessitant pas d'investissements supplémentaires.

L'invention concerne aussi un système de traitement d'un flux principal de gaz circulant dans un conduit principal, ledit système étant remarquable en ce qu'il comprend :
- un conduit de prélèvement, dans le conduit principal, d'un flux secondaire de gaz,
- une unité d'épuration du flux secondaire de gaz de sorte à produire, d'une part, un Gaz Naturel biologique pour Véhicule, et, d'autre part, des gaz résiduels, et
- un conduit d'injection des gaz résiduels issus de l'épuration dans le conduit principal de sorte à obtenir un flux principal dans lequel ont été injectés les gaz résiduels.

Le système selon l'invention est avantageusement autonome et peut aisément être intégré dans une installation existante entre un méthaniseur et une ou plusieurs unités de valorisation du gaz, par exemple de type moteur, turbine, chaudière, etc.

Avantageusement, le système est configuré pour traiter un flux principal de gaz en entrée du conduit principal constitué de gaz produit par méthanisation (biogaz) et/ou de gaz produit par méthanation ou pyrogazéification (gaz de synthèse).

De préférence, l'unité d'épuration du flux secondaire de gaz est configurée pour fonctionner à un rendement épuratoire inférieur à 90 %, de préférence en permanence. En d'autres termes, le rendement épuratoire de l'unité d'épuration ne peut dépasser les 90 %. De préférence, le rendement épuratoire de l'unité d'épuration est maintenu entre 75 et 85 %.

De manière avantageuse, le système comprend une unité de production telle que, par exemple une unité de méthanisation pour la production de biogaz et/ou une unité de production d'un gaz de synthèse, par exemple par méthanation ou par pyrogazéification.

Dans une forme de réalisation, le système comprend une unité de prétraitement, permettant par exemple d'éliminer les polluants du flux principal de gaz produit à la sortie de l'unité de production.

Selon un aspect de l'invention, le système comprend au moins une unité de valorisation du flux principal de gaz dans lequel ont été injectés les gaz résiduels. Une telle unité de valorisation peut par exemple se présenter sous la forme d'un moteur, d'une turbine, d'une chaudière, d'un module de cogénération, ou de tout autre type de module adapté.

### PRESENTATION DES FIGURES

L'invention sera mieux comprise à la lecture de la description qui va suivre, donnée uniquement à titre d'exemple, et se référant aux dessins annexés sur lesquels :
- la figure 1 illustre schématiquement une forme de réalisation du système selon l'invention,
- la figure 2 illustre schématiquement un mode de réalisation du procédé selon l'invention.

Il faut noter que les figures exposent l'invention de manière détaillée pour mettre en oeuvre l'invention, lesdites figures pouvant bien entendu servir à mieux définir l'invention le cas échéant.

### DESCRIPTION D'UN OU PLUSIEURS MODES DE REALISATION ET DE MISE EN OEUVRE

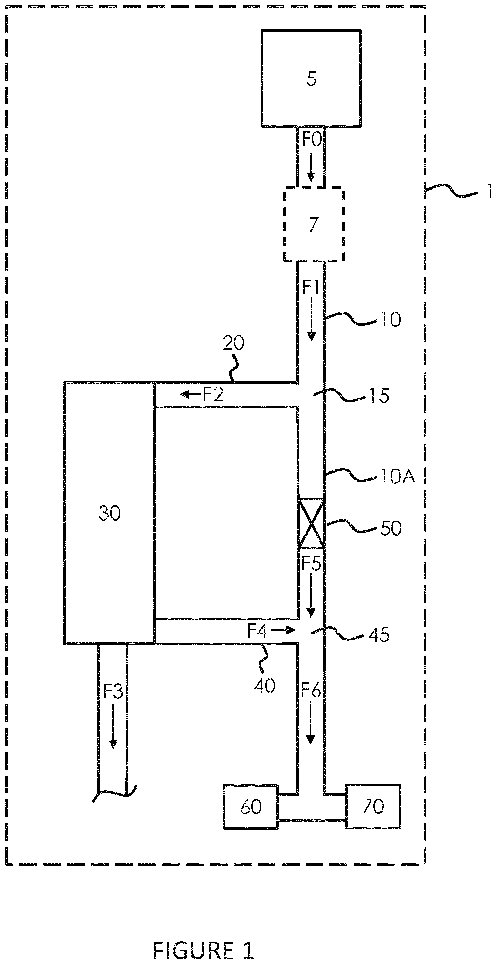

Le système de traitement selon l'invention va maintenant être décrit en référence à la figure 1. Ce système 1 s'applique particulièrement au traitement d'un biogaz produit par méthanisation ou au traitement d'un gaz de synthèse produit par méthanation ou par pyrogazéification. Le gaz traité dans le système 1 peut aussi éventuellement avoir subi un prétraitement. Le gaz traité peut être destiné à être stocké ou valorisé de manière connue dans une unité de valorisation, par exemple de type moteur, turbine, chaudière, module de cogénération, etc.

Le système 1 selon l'invention permet de produire du Gaz Naturel biologique pour Véhicule (ou BioGNV) par épuration tout en permettant le recyclage des gaz résiduels issus de l'épuration dans le flux principal de gaz avant stockage ou valorisation.

A cette fin, le système 1 comprend tout d'abord un conduit principal 10 dans lequel circule un flux principal F1 de gaz produit par une unité de production 5, par exemple une unité de méthanisation, de méthanation ou de pyrogazéification. Dans cet exemple, non limitatif, le système 1 comprend en outre, entre l'unité de production 5 et le conduit principal 10, un module de prétraitement 7 dont la fonction est d'éliminer les polluants tels que l'hydrogène sulfuré (H₂S), les composés organiques volatiles (COV) tels que, par exemple, les siloxanes, et de sécher le gaz (élimination d'une fraction de l'eau). Ce prétraitement permet, d'une part, de respecter les normes d'émission des fumées dans l'atmosphère (par exemple en abattant le taux d'hydrogène sulfuré dans le gaz on réduit le taux de SO₂ dans les fumées) et, d'autre part, de protéger les équipements (chaudière, moteur, etc) de la corrosion (l'hydrogène sulfuré et l'eau formant de l'acide) et de l'encrassement (les siloxanes lors de la combustion formant de la silice qui se dépose sur les parties internes des équipements (pistons et soupapes des moteurs par exemple) et provoque leur encrassement voire leur casse).

Le système 1 comprend ensuite un conduit de prélèvement 20, une unité d'épuration 30 et un conduit d'injection 40. Le conduit de prélèvement 20 est relié, d'une part, au conduit principal 10 en une zone de raccordement amont 15, et, d'autre part, à l'unité d'épuration 30 et permet de prélever une fraction de gaz (ou flux secondaire F2) dans le flux principal F1 circulant dans le conduit principal 10 afin de l'acheminer jusqu'à l'unité d'épuration 30. Le conduit de prélèvement 20, l'unité d'épuration 30 et le conduit d'injection 40 constituent un bras de dérivation d'une partie du gaz circulant dans le conduit principal 10. Autrement dit, la partie du système 1 comprenant le conduit de prélèvement 20, l'unité d'épuration 30 et le conduit d'injection 40 est connectée en parallèle d'une portion 10 A du conduit principal 10.

Le débit de prélèvement du flux secondaire F2 prélevé dans le flux principal F1 est réglé par l'unité d'épuration 30 à l'aide d'un moyen adapté (non représenté). Ce moyen peut être une vanne mais également un module de régulation contrôlant la fréquence de rotation d'un surpresseur, d'un compresseur ou d'une pompe à vide selon la technologie d'épuration mise en oeuvre et des pressions de travail utilisées dans l'unité d'épuration 30, ou tout autre moyen adapté. La régulation du débit de prélèvement d'un flux secondaire F2 dans un flux principal F1 par une unité d'épuration 30 étant connue en soi, il ne sera pas davantage détaillé ici.

L'unité d'épuration 30 permet d'épurer le gaz du flux secondaire F2 prélevé pour en augmenter la teneur en méthane (CH₄), par exemple jusqu'à une valeur de l'ordre de 97 %, afin de le transformer en un flux de BioGNV F3. Une telle épuration produit des gaz résiduels qui, selon l'invention, vont être réinjectés dans le flux principal F1 de gaz. Ces gaz résiduels comprennent du dioxyde de carbone (CO₂) et du méthane dont la proportion augmente avec la diminution du rendement épuratoire de l'unité d'épuration 30.

Dans cet exemple non limitatif, le conduit d'injection 40 relie l'unité d'épuration 30 au conduit principal 10 en une zone de raccordement aval 45 du conduit principal 10 afin de permettre le retour du flux de gaz résiduels F4 produits lors de l'épuration du gaz dans le flux principal F1 de gaz circulant dans le conduit principal 10. Une vanne déprimogène 50 permet d'assurer la circulation du flux principal F1 /F5 dans le conduit principal 10. En variante, tout autre moyen de circulation du gaz peut être utilisé selon la technologie d'épuration mise en oeuvre, comme cela a été décrit précédemment. Ainsi, par exemple, l'unité d'épuration 30 peut comporter un surpresseur, ou un compresseur ou une pompe à vide qui permet de soutirer le flux secondaire F2 et de réinjecter le flux de gaz résiduels F4 dans le conduit principal 10.

On notera de plus que les gaz résiduels peuvent ne pas être nécessairement injectés en aval dans le flux principal de gaz. Par exemple, les gaz résiduels peuvent être également injectés en amont du conduit de prélèvement 20, par exemple dans le ciel gazeux de l'unité de production 5 ou bien en aval de l'unité de prétraitement 7 selon le type d'installation sur laquelle on installe l'unité de production de BioGNV.

Selon un aspect de l'invention, le rendement épuratoire de l'unité d'épuration 30 est volontairement limité à 90 %, de préférence à une valeur comprise entre 75 et 85 %. Un tel rendement permet avantageusement de réduire significativement à la fois les coûts de fonctionnement (énergie électrique) et les coûts de réalisation de l'unité d'épuration 30 tout en permettant la production de BioGNV et le recyclage des gaz résiduels dont le taux de méthane est relativement élevé, pour éviter qu'ils ne se perdent. A cette fin, l'unité d'épuration 30 peut être conçue de sorte que son rendement épuratoire soit compris dans l'intervalle souhaité, par exemple entre 75 et 85 %, toujours dans un souci de réduction des coûts et de recyclage du méthane contenu dans les gaz résiduels.

Le système 1 selon l'invention est ainsi particulièrement adapté aux exploitations agricoles qui recyclent leurs matières organiques dans des méthaniseurs pour produire du biogaz. Ce biogaz peut être par exemple avantageusement utilisé à la fois pour produire de l'énergie électrique ou thermique et du BioGNV pour leurs véhicules. Dans l'exemple non limitatif de la figure 1, le système 1 comprend un moteur 60 permettant de produire de l'énergie électrique et une chaudière 70 permettant de produire de l'énergie thermique. Un tel moteur 60 et une telle chaudière 70 étant connus en soi, de même que leur fonctionnement, ils ne seront pas davantage détaillés ici.

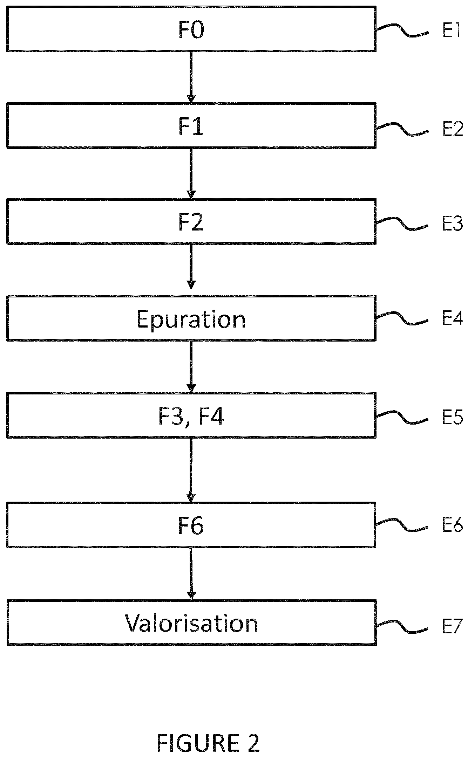

L'invention va maintenant être décrite dans sa mise en oeuvre en référence aux figures 1 et 2.

Tout d'abord, dans une étape E1, l'unité de production 5 produit un flux F0 de gaz, par exemple par méthanisation de matières organiques. Ce flux F0 est ensuite prétraité, dans une étape E2, en un flux principal F1 de gaz par le module de prétraitement 7 afin d'être acheminé par le conduit principal 10.

Un flux secondaire F2 de gaz est ensuite prélevé, dans une étape E3, dans le flux principal F1 circulant dans le conduit principal 10. Ce flux secondaire F2 est acheminé par le conduit de prélèvement 20 jusqu'à l'unité d'épuration 30 qui l'épure, dans une étape E4, à un rendement épuratoire inférieur à 90 % de sorte à produire, dans une étape E5, d'une part, un flux F3 de Gaz Naturel biologique pour Véhicule, et, d'autre part, un flux F4 de gaz résiduels, dont la teneur en méthane est de préférence comprise entre 10 et 25%.

Dans une étape E6, les gaz résiduels issus de l'épuration sont injectés dans le flux principal de gaz F5 ayant passé la vanne déprimogène 50 via le conduit d'injection 40 afin de constituer un flux principal F6 dans lequel ont été injectés les gaz résiduels et dont la composition de méthane est supérieure à 40 % afin de pouvoir être valorisé, c'est à dire utilisé, dans le moteur 60 et dans la chaudière 70 dans une étape E7.

Comme indiqué précédemment, on notera que dans un autre mode de réalisation, les gaz résiduels pourraient être injectés en amont du conduit de prélèvement 20. La seule contrainte est que le gaz issu du mélange avec le flux F4 de gaz résiduel ait une teneur en méthane suffisante pour permettre le fonctionnement des équipements de valorisation du gaz (ici le moteur 60 et la chaudière 70). Cette teneur peut être d'environ 40 % mais il est important de préciser que cette valeur dépend des constructeurs d'équipements de valorisation et destruction de gaz et pourra évoluer dans le futur au grès des évolutions technologiques.

Un exemple va maintenant être donné à l'aide de tables numériques dont les données sont issues d'un test effectué sur un système 1 comprenant une unité de production 5 de type méthaniseur mais qui est dépourvu d'unité de prétraitement 7 et dont l'unité d'épuration 30 présente un rendement épuratoire de 80 %.

Le débit du flux principal de gaz F1 en entrée du conduit principal 10 est de 100 Nm³/h avec une proportion de 55 % de méthane (CH₄) et 45 % de dioxyde de carbone (CO₂), respectivement avec un débit de 55,0 Nm³/h et 45,0 Nm³/h :

**Table 1**

| **Flux F1** | | |
|---|---|---|
| **Composition** | **(%)** | **Débit** |
| CH₄ | 55 % | 55,0 Nm³/h |
| CO₂ | 45 % | 45,0 Nm³/h |
| **Total** | **100 %** | **100,0 Nm³/h** |

Le débit du flux secondaire F2 de gaz prélevé dans le conduit principal 10 est de 45 Nm³/h. Le gaz soutiré présente toujours une proportion de 55 % de méthane (CH₄) et 45 % de dioxyde de carbone (CO₂), respectivement avec un débit de 24,8 Nm³/h et 20,3 Nm³/h :

**Table 2**

| **Flux F2** | | |
|---|---|---|
| **Composition** | **(%)** | **Débit** |
| CH₄ | 55 % | 24,8 Nm³/h |
| CO₂ | 45 % | 20,3 Nm³/h |
| **Total** | **100 %** | **45,0 Nm³/h** |

Le débit du flux principal F5 dans le conduit principal 10 après soutirage est de 55 Nm³/h avec également une proportion de 55 % de méthane (CH₄) et 45 % de dioxyde de carbone (CO₂), respectivement avec un débit de 30,3 Nm³/h et 24,8 Nm³/h :

**Table 3**

| **Flux F5** | | |
|---|---|---|
| **Composition** | **(%)** | **Débit** |
| CH₄ | 55 % | 30,3 Nm³/h |
| CO₂ | 45 % | 24,8 Nm³/h |
| **Total** | **100 %** | **55,0 Nm³/h** |

Le débit du flux de BioGNV F3 produit par l'unité d'épuration 30 est de 100 Nm³/h avec une proportion de 97 % de méthane (CH₄) et 3 % de dioxyde de carbone (CO₂), respectivement avec un débit de 19,8 Nm³/h et 0,6 Nm³/h :

**Table 4**

| **Flux F3** | | |
|---|---|---|
| **Composition** | **(%)** | **Débit** |
| CH₄ | 97 % | 19,8 Nm³/h |
| CO₂ | 3 % | 0,6 Nm³/h |
| **Total** | **100 %** | **20,4 Nm³/h** |

Le débit du flux de gaz résiduels F4 produit par l'unité d'épuration 30 est de 24,6 Nm³/h avec une proportion de 20 % de méthane (CH₄) et 80 % de dioxyde de carbone (CO₂), respectivement avec un débit de 5 Nm³/h et 19,6 Nm³/h :

**Table 5**

| **Flux F4** | | |
|---|---|---|
| **Composition** | **(%)** | **Débit** |
| CH₄ | 20 % | 5,0 Nm³/h |
| CO₂ | 80 % | 19,6 Nm³/h |
| **Total** | **100 %** | **24,6 Nm³/h** |

Enfin, le débit du flux principal de gaz F6 après injection des gaz résiduels est de 79,6 Nm³/h avec une proportion de 44 % de méthane (CH₄) et 56 % de dioxyde de carbone (CO₂), respectivement avec un débit de 35,2 Nm³/h et 44,4 Nm³/h :

**Table 6**

| **Flux F6** | | |
|---|---|---|
| **Composition** | **(%)** | **Débit** |
| CH₄ | 44 % | 35,2 Nm³/h |
| CO₂ | 56 % | 44,4 Nm³/h |
| **Total** | **100 %** | **79,6 Nm³/h** |

La proportion de méthane est ainsi supérieure à 40 %, ce qui permet avantageusement d'utiliser le flux principal de gaz sortant (dans lequel ont été injectés les gaz résiduels) dans une unité de valorisation de type moteur 60 ou chaudière 70. Le système 1 selon l'invention s'avère simple et efficace et permet à la fois de produire du BioGNV à bas coût tout en recyclant les gaz résiduels issus de la production dudit BioGNV et dont le taux de méthane est relativement élevé afin de les valoriser pour éviter de les perdre.

## Revendications

1. Procédé de traitement d'un flux principal (F1) de gaz, ledit procédé étant **caractérisé en ce qu'**il comprend :
- une étape (E3) de prélèvement, dans le flux principal (F1), d'un flux secondaire (F2) de gaz,
- une étape (E4) d'épuration du flux secondaire (F2) de gaz de sorte à produire, d'une part, un Gaz Naturel biologique pour Véhicule (F3), et, d'autre part, des gaz résiduels (F4), et
- une étape (E6) d'injection des gaz résiduels (F4) issus de l'épuration dans le flux principal (F5) de gaz.

2. Procédé selon la revendication 1, dans lequel le flux principal (F1) de gaz est constitué d'un biogaz issu d'une méthanisation et/ou d'un gaz de synthèse issu d'une méthanation ou d'une pyrogazéification.

3. Procédé selon l'une des revendications 1 et 2, dans lequel l'épuration (E4) du flux secondaire (F2) de gaz est réalisée à un rendement épuratoire inférieur à 90 %.

4. Procédé selon la revendication 3, dans lequel l'épuration (E4) du flux secondaire (F2) de gaz est réalisée à un rendement épuratoire compris entre 75 et 85 %.

5. Procédé selon l'une des revendications 3 et 4, dans lequel l'injection (E6) des gaz résiduels (F4) issus de l'épuration (E4) dans le flux principal (F5) de gaz est réalisée de sorte que le taux de méthane dans le flux principal (F6) après injection soit supérieur à 40%.

6. Système (1) de traitement d'un flux principal (F1) de gaz circulant dans un conduit principal (10), ledit système (1) étant **caractérisé en ce qu'**il comprend :
- un conduit (20) de prélèvement, dans le conduit principal (10), d'un flux secondaire (F2) de gaz,
- une unité (30) d'épuration du flux secondaire (F2) de gaz de sorte à produire, d'une part, un Gaz Naturel biologique pour Véhicule (F3), et, d'autre part, des gaz résiduels (F4), et
- un conduit (40) d'injection des gaz résiduels (F4) issus de l'épuration dans le conduit principal (10) de sorte à obtenir un flux principal (F6) dans lequel ont été injectés les gaz résiduels.

7. Système (1) selon la revendication 6, ledit système (1) étant configuré pour traiter un flux principal (F1) de gaz en entrée du conduit principal (10) constitué de gaz produit par méthanisation et/ou de gaz produit par méthanation ou pyrogazéification.

8. Système (1) selon l'une des revendications 6 et 7, dans lequel l'unité d'épuration (30) du flux secondaire (F2) de gaz est configurée pour fonctionner à un rendement épuratoire inférieur à 90 %.

9. Système (1) selon la revendication 8, dans lequel l'unité d'épuration (30) est configurée pour fonctionner en permanence à un rendement épuratoire inférieur à 90%.

10. Système (1) selon l'une des revendications 8 et 9, dans lequel le rendement épuratoire de l'unité d'épuration est maintenu entre 75 et 85 %.
